# EUROPÄISCHE PATENTSCHRIFT

(11) **EP 1 683 487 B1**
(45) Veröffentlichungstag und Bekanntmachung des Hinweises auf die Patenterteilung: **27.02.2008**
(21) Anmeldenummer: 05028335.7
(22) Anmeldetag: 23.12.2005
(51) Int. Cl.: A61B 17/00, A61B 17/04

(54) **Einrichtung zum chirurgischen Verschluss eines Trokardurchstichs**
Device for closing a trocar puncture wound
Dispositif de fermeture d'une blessure à perforation à trocart

(30) Priorität: 24.01.2005 AT 952005
(43) Veröffentlichungstag der Anmeldung: 26.07.2006
(73) Patentinhaber: AMI Agency for Medical Innovations GmbH, 6800 Feldkirch (AT)
(72) Erfinder: Bischof, Georg, Dr., 1180 Wien (AT); Wild, Thomas, Dr., 1200 Wien (AT); Unger, Ewald, Ing., 1210 Wien (AT)
(74) Vertreter: Hofmann, Ralf U.

(56) Entgegenhaltungen:
- US-A- 5 591 180
- US-A1- 2002 016 614

## Beschreibung

Die Erfindung betrifft eine Einrichtung zum chirurgischen Verschluss eines bei einer laparoskopischen Operation eingebrachten Trokardurchstichs durch eine posteriore Fascie, welche einen Tubus mit einem vorderen Einsteckende und einem hinteren Ende aufweist, in dessen Mantelwand gegenüberliegende Fensteröffnungen ausgebildet sind, in die an ihren vom Einsteckende des Tubus abgelegenen Rändern jeweils mindestens eine in der Mantelwand in Längsrichtung des Tubus verlaufende Bohrung mündet, in welcher jeweils eine Nadel der Einrichtung zwischen einer die jeweilige Fensteröffnung freigebenden zurückgezogenen Stellung und einer die Fensteröffnung überquerenden Endstellung verschiebbar ist.

Zur Durchführung von laparoskopischen Operationen werden Trokare durch Durchstiche durch die Bauchdecke in den Bauchraum eingeführt. Der Bauchraum wird innen durch das Bauchfell (Peritoneum) begrenzt. Nach außen schließt eine Fascie an, die als posteriore Fascie bezeichnet wird und für die Stabilität der Bauchdecke von wesentlicher Bedeutung ist. Nach außen schließt eine Muskelschicht an, auf welche wiederum eine Fascie, eine Fettschicht und schließlich die Haut folgen.

Wenn ein Trokar am Ende der Operation aus dem Bauchraum herausgezogen wird, so sollte ab einem Durchmesser des Trokars von 10mm die posteriore Fascie verschlossen werden, um Stichkanalhernien vorzubeugen. Da der Stichkanal nach dem Herausziehen des Trokars zusammenfällt, muss hierzu das Gewebe mit Pinzetten auseinandergezogen werden und mit einem Nadelhalter die Nadel in den Bereich der posterioren Fascie eingeführt werden und das Gewebe zum Setzen einer Naht entsprechend durchstochen werden. Dieser Vorgang ist auch aufgrund der mangelnden Sicht schwer durchzuführen und wird umso schwieriger, je dicker die Bauchdecke des Patienten ist.

Um das Setzen einer Fasciennaht zu erleichtern, ist bereits eine Hilfseinrichtung bekannt, welche von einem Konus gebildet wird, der zwei schräg zur Längsrichtung verlaufende, sich kreuzende Bohrungen aufweist. Der Konus wird soweit in den Stichkanal eingeführt, dass die Eingänge in die beiden Bohrungen außerhalb der Bauchdecke liegen und die Ausgänge der Bohrungen an den vorgesehenen Einstichstellen für die Nadeln liegen. Die Nadel mit dem daran angeordneten Faden wird durch eine der Bohrungen durchgeführt und am Ausgang der Bohrung durch das Gewebe in den Bauchraum durchgestochen. Der Faden wird von der Nadel freigegeben. Die Nadel wird durch die zweite Bohrung durchgestochen und der Faden mit einem Greifer, der durch ein anderes, dünneres Trokar eingeführt ist, in die Nadel eingelegt, worauf die Nadel wiederum zurückgezogen wird. Nachteilig ist es hierbei unter anderem, dass während dieses Vorgangs das Pneumoperithoneum aufrechterhalten werden muss. Weiters muss die Optik, um den Vorgang zu kontrollieren, auf ein dünneres Trokar gewechselt werden (für welches kein Fascienverschluss benötigt wird). Auch besteht beim Durchstechen der Nadel in den Bauchraum ein Verletzungsrisiko für den Patienten.

In der Praxis wird aufgrund der vorhandenen Schwierigkeiten ein Fascienverschluss häufig nicht durchgeführt, mit den damit verbundenen Risiken für den Patienten.

Eine Einrichtung der eingangs genannten Art ist aus der US 5,591,180 A bekannt. Bei dieser Einrichtung muss der Faden bereits vor dem Einführen des Instruments durch den Trokardurchstich an zwei Hülsen angebracht werden, die ihrerseits in Nuten im Bereich der distalen Ränder der Fensteröffnungen in der Mantelwand des Tubus angeordnet werden müssen. Zum Vernähen des Gewebedurchstichs werden die Nadeln mit Hilfe von Schiebern achsial in den Schlitzen verschoben, wobei sie beim Übergang aus der zurückgezogenen Stellung in die vordere Stellung das durch die Fensteröffnungen hindurchtretende Gewebe durchstechen. Erreichen die Nadelspitzen die distalen Ränder der Fenster, so erfolgt ein Einstich der Nadelspitzen in die dort angeordneten Hülsen. Dabei bilden die Nadelspitzen mit den Hülsen einen Reibschluss derart, dass beim Zurückziehen der Nadeln in die zurückgezogene Stellung die Hülsen zusammen mit dem Faden durch das zu vernähende Gewebe hindurchgezogen werden. Die beiden Hälften des zweigeteilten Tubus werden sodann aus dem Trokardurchstich herausgezogen und auseinandergenommen. Dabei wird die Fadenschlinge, die zunächst durch den inneren Zentralkanal des Instruments nach außen geragt ist, durch den Trokardurchstich hindurch in das Körperinnere gezogen und durch weiteres Anziehen an diesen Fadenabschnitten des Fadens wird die Wunde schließlich verschlossen.

Die US 2002/0016614 A1 offenbart eine gattungsfremde Einrichtung zum Vernähen von Öffnungen in einer Arterienwand oder dergleichen.

Aufgabe der Erfindung ist es, eine verbesserte Einrichtung der eingangs genannten Art bereitzustellen, mit der eine einfache und sichere Fascien-Nahtvorlage durchführbar ist. Erfindungsgemäß gelingt dies durch eine Einrichtung mit den Merkmalen des Patentanspruchs 1.

Um mit einer erfindungsgemäßen Einrichtung eine Fasciennaht zu setzen, wird der Tubus mit seinem vorderen Einsteckende voraus so tief im Stichkanal des Trokars angeordnet, dass sich die Fensteröffnungen in der Mantelwand an der Stelle befinden, an der das Gewebe zu durchstechen ist. Aufgrund der Eigenelastizität des Gewebes steht dieses im Bereich der Fensteröffnungen etwas in den Tubus hinein, wobei dies gegebenenfalls auch durch einen Unterdruck im Tubus oder durch mechanisches Hineindrücken oder -ziehen (beispielsweise von außen manuell oder von innen mittels einer Pinzette) noch verstärkt werden kann. In der Folge werden die Hohlnadeln aus ihren die Fensteröffnungen freigebenden zurückgezogenen Stellungen in ihre die Fensteröffnungen überquerende Endstellungen verschoben, wobei das in die Fensteröffnungen eingefallene Gewebe durchstochen wird. In den vorgeschobenen Endstellungen der Hohlnadeln wird ein Führungskanal zum Durchführen eines Fadeneinzugselements ausgebildet, der durch die inneren Kanäle der Hohlnadeln und durch den die vorderen Enden der inneren Kanäle der Hohlnadeln verbindenden gekrümmten Verbindungsabschnitt verläuft.

Nachdem der Faden mittels des Fadeneinzugselements in den Führungskanal gezogen worden ist und sich durch diesen erstreckt, werden die Hohlnadeln wieder in ihre die Fensteröffnungen freigebenden zurückgezogenen Stellungen zurückgeschoben. Der Tubus kann nunmehr aus dem Stichkanal herausgezogen werden, wobei der sich im Verbindungsabschnitt des Führungskanals befindende Abschnitt des Fadens quer zur jeweiligen lokalen Längserstreckung des Verbindungsabschnitts aus diesem herausgebracht wird. Beispielsweise ist hierzu der Führungskanal im Verbindungsabschnitt zum den Tubus umgebenden Außenraum hin durch einen durchreißbaren Streifen mit einer entsprechend dünnen Wandstärke begrenzt. Die in den Hohlnadeln sich befindenden Abschnitte des Fadens werden beim Zurückziehen des Tubus aus diesen herausgezogen (durch die an den Rändern der Fensteröffnungen liegenden Mündungen der inneren Kanäle der Hohlnadeln). Der nunmehr beidseitig des Stichkanals des Trokars durch die zu verschließende posteriore Fascie verlaufende Faden kann in der Folge zur Naht verknüpft werden.

Denkbar und möglich ist es auch, dass der Verbindungsabschnitt des Führungskanals über seine gesamte Länge durch einen Schlitz mit dem den Tubus umgebenden Außenraum verbunden ist. Das Fadeneinzugselement muss dann eine Dicke aufweisen, die größer als die Breite des Schlitzes ist, um die Führung durch den Verbindungsabschnitt des Führungskanals sicherzustellen. Vorzugsweise ist aber eine Verschlusseinrichtung für den Verbindungsabschnitt des Führungskanals vorhanden.

In einer möglichen Ausführungsform der Erfindung ist der Tubus an seinem vorderen Einsteckende geschlossen. Der Verbindungsabschnitt kann in diesem Fall durch die die vordere Stirnseite des Tubus bildende Stirnwand verlaufen.

In einer anderen möglichen Ausführungsform der Erfindung kann der Tubus an seinem Einsteckende offen ausgebildet sein, wobei die Einrichtung gleichzeitig als Trokar ausgebildet ist. Der gekrümmte Verbindungsabschnitt des Führungskanals kann in diesem Fall in der Mantelwand des Tubus im Bereich vor den Fensteröffnungen verlaufen.

Vorzugsweise wird der Verbindungsabschnitt des Führungskanals von einer an der Außenseite des Tubus eingebrachten Nut gebildet, die zunächst von einer Verschlusseinrichtung verschlossen ist. In der vorderen Endstellung der Hohlnadeln kann somit ein durchgängiger, über seine gesamte Länge geschlossener Führungskanal ausgebildet werden. Zum Herausbringen des Fadens aus dem Verbindungsabschnitt wird dann die Verschlusseinrichtung geöffnet. Dies kann beispielsweise durch ein Durchreißen einer die Nut im Tubus überdeckenden, am Tubus beidseitig der Nut befestigten (insbesondere durch Verkleben) streifenförmigen Folie durchgeführt werden, welche in diesem Fall die Verschlusseinrichtung bildet. Denkbar und möglich wäre es auch, dass eine die Nut im Tubus überdeckende streifenförmige Folie auf einer Seite der Nut nur mit einer solchen Haftkraft am Tubus angeklebt ist, dass die Folie beim Zurückziehen des Tubus aus dem Stichkanal einseitig abreißbar ist und der Faden somit aus der Nut freigegeben wird.

Zur gleichzeitigen Verschiebung der Hohlnadeln zwischen ihren die Fensteröffnungen freigebenden zurückgezogenen Stellungen und ihren die Fensteröffnungen überquerenden Endstellungen ist vorteilhafterweise eine Verschiebeeinrichtung vorgesehen. Hierzu kann in einer vorteilhaften Ausführungsform der Erfindung ein verdrehbarer Betätigungsring vorhanden sein, der über eine Kulissenführung bei seiner Verdrehung einen die Hohlnadeln haltenden Nadelhaltering in Längsrichtung des Tubus verschiebt.

Weitere Vorteile und Einzelheiten der Erfindung werden im Folgenden anhand der beiliegenden Zeichnung erläutert. In dieser zeigen:
- Fig. 1: eine Schrägsicht einer erfindungsgemäßen Einrichtung gemäß einem ersten Ausführungsbeispiel der Erfindung in den zurückgezogenen Stellungen der Hohlnadeln;
- Fig. 2: die Einrichtung von Fig. 1, wobei Teile der Einrichtung nach Art einer Explosionsdarstellung auseinandergezogen dargestellt sind;
- Fig. 3: eine vergrößerte Darstellung der Verschlusseinrichtung für den Verbindungsabschnitt des Führungskanals, in Schrägsicht;
- Fig. 4: eine vergrößerte Darstellung des vorderen Endabschnitts der Einrichtung, in Schrägsicht;
- Fig. 5: eine Darstellung analog Fig. 4, aber ohne die Verschlusseinrichtung des Verbindungsabschnitts des Führungskanals;
- Fig. 6: eine Darstellung entsprechend Fig. 5, aber in der vorgeschobenen Endstellung der Hohlnadeln;
- Fig. 7: einen Längsschnitt durch die Einrichtung, in der zurückgezogenen Stellung der Hohlnadeln, wobei die Gewebeschichten der Bauchdecke schematisch angedeutet sind;
- Fig. 8: eine Darstellung entsprechend Fig. 7, aber in einer Zwischenstellung der Hohlnadeln und mit einem in den Tubus eingeführten Endoskop;
- Fig. 9: eine Darstellung entsprechend Fig. 7, aber in der vorgeschobenen Endstellung der Hohlnadeln;
- Fig. 10: einen vergrößerten Ausschnitt von Fig. 9;
- Fig. 11: eine Darstellung entsprechend Fig. 9, aber mit einem durch den Führungskanal durchgeschobenen Fadeneinzugselement, an dem ein Faden angeordnet ist;
- Fig. 12: eine Darstellung entsprechend Fig. 11, aber mit dem durch den Führungskanal durchgezogenen Faden;
- Fig. 13: den vorderen Abschnitt der Einrichtung in Schrägsicht und die Bauchdecke im schematischen Schnitt, nach dem Herausziehen des Tubus aus dem Stichkanal;
- Fig. 14: eine Schrägsicht einer zweiteiligen Ausführungsform einer Verschlusseinrichtung für den Verbindungsabschnitt des Führungskanals;
- Fig. 15: eine Explosionsdarstellung einer weiteren Ausführungsform einer erfindungsgemäßen Einrichtung;
- Fig. 16: eine vergrößerte Darstellung des Verschlusselements für den Verbindungsabschnitt des Führungskanals, in Schrägsicht;
- Fig. 17: einen vorderen Endabschnitt der Einrichtung gemäß dieser weiteren Ausführungsform in der vorgeschobenen Endstellung der Hohlnadeln, ohne die Verschlusseinrichtung;
- Fig. 18: eine Explosionsdarstellung noch einer weiteren Ausführungsform einer erfindungsgemäßen Einrichtung;
- Fig. 19: und Fig. 20 Darstellungen der Endabschnitte der Einrichtung gemäß dieser Ausführungsform in zwei Stellungen des Schneidwerkzeugs;
- Fig. 21: eine schematische Schrägsicht eines vorderen Endabschnitts gemäß noch einer weiteren Ausführungsform einer erfindungsgemäßen Einrichtung;
- Fig. 22: eine Blockdarstellung der elektronischen Auswerteinheit;
- Fig. 23: eine schematische Darstellung einer möglichen Ausführung eines Gegenlagers für die jeweilige Hohlnadel und
- Fig. 24: eine schematische Darstellung einer weiteren Ausführungsform einer Hohlnadel.

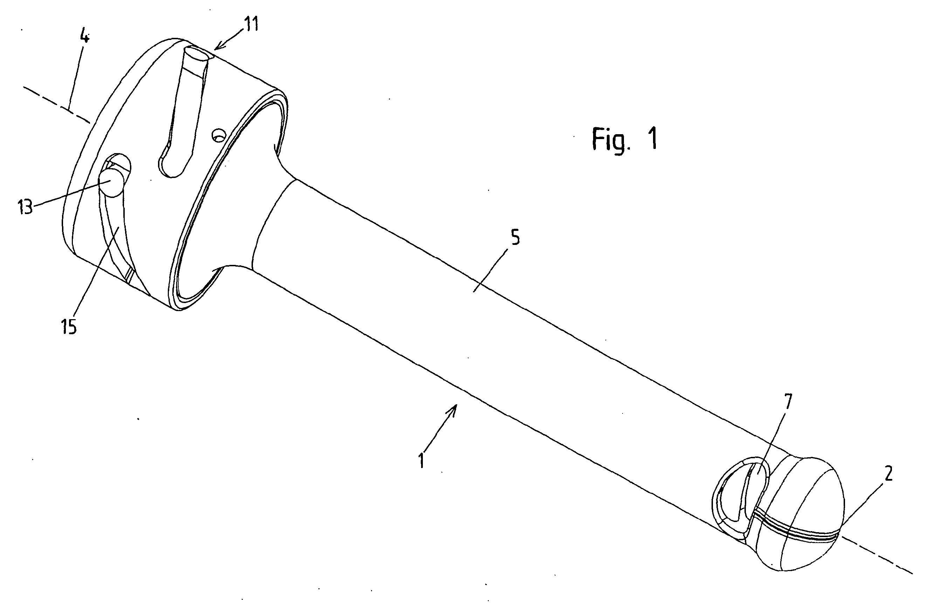

Eine erste Ausführungsform einer erfindungsgemäßen Einrichtung ist in den Fig. 1 bis 13 dargestellt. Die Einrichtung weist einen Tubus 1 (= eine Hülse) auf, der vorzugsweise aus Kunststoff besteht, beispielsweise wie er für Trokare verwendet wird, und ein vorderes Einsteckende 2 und ein hinteres Ende 3 besitzt. Beim Einsatz der Einrichtung ist der Tubus 1 mit dem Einsteckende voraus in einen Stichkanal eines Trokars einzuführen.

Wenn im Rahmen dieser Schrift von "vorne" und "hinten" die Rede ist, so ist hiermit die Lage in Bezug auf das Einsteckende 2 gemeint, d. h. ein weiter vorne liegendes Teil befindet sich näher beim Einsteckende als ein weiter hinten liegendes Teil.

Der Tubus 1 besitzt eine im Querschnitt gesehen ringförmige Mantelwand 5, die einen inneren, in Längsrichtung des Tubus verlaufenden Hohlraum 6 umgibt. Der Tubus 1 ist an seinem hinteren Ende 3 offen. In diesem Ausführungsbeispiel ist der Tubus 1 an seinem vorderen Einsteckende geschlossen.

In der Mantelwand 5 des Tubus 1 sind zwei gegenüberliegende Fensteröffnungen 7 ausgebildet, durch die der innere Hohlraum 6 des Tubus mit dem den Tubus 1 umgebenden Außenraum verbunden ist. Die seitlichen Fensteröffnungen 7 liegen somit quer zur Längsachse 4 des Tubus 1.

Bevorzugterweise befinden sich die gegenüberliegenden Fensteröffnungen 7 in einem vorderen Endabschnitt des Tubus 1, günstigerweise zumindest im vorderen Viertel der Längserstreckung des Tubus 1.

Von den Fensteröffnungen 7 wird vorzugsweise jeweils mindestens 30% des Umfangs des Tubus freigegeben (an der breitesten Stelle der jeweiligen Fensteröffnung). In Längsrichtung des Tubus beträgt die Größe der jeweiligen Fensteröffnung an ihrer breitesten Stelle günstigerweise mindestens 10mm. Der Bereich von 10 bis 30mm ist bevorzugt.

In der Mantelwand 5 des Tubus 1 verlaufen in Längsrichtung des Tubus sich erstreckende, parallel zur Längsachse 4 des Tubus 1 liegende Bohrungen 8. In diesem Ausführungsbeispiel mündet in die jeweilige Fensteröffnung 7 jeweils eine Bohrung 8, und zwar am vom vorderen Einsteckende 2 entfernt gelegenen Rand der Fensteröffnung 7.

Die Bohrungen 8 erstrecken sich ausgehend von den Fensteröffnungen 7 in der Mantelwand 5 nach hinten, und zwar über die gesamte Länge des Abschnitts des Tubus 1, in welchem dieser einen konstanten Innendurchmesser aufweist.

Am hinteren Ende dieses Abschnitts schließt in diesem Ausführungsbeispiel ein Abschnitt 9 mit einem vergrößerten Innen- und auch Außendurchmesser an. Die Bohrungen 8 münden an der nach hinten gerichteten Stufe zwischen diesen beiden Abschnitten des Tubus 1.

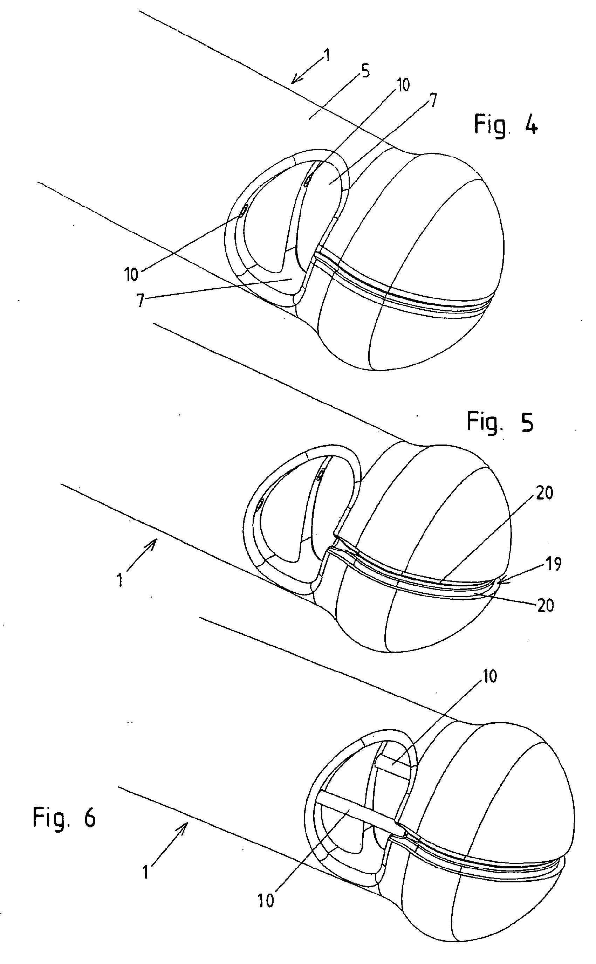

In den Bohrungen 8 ist jeweils eine Hohlnadel 10 verschiebbar geführt. Die Hohlnadeln 10 sind hierbei zwischen einer die jeweilige Fensteröffnung 7 freigebenden, zurückgezogenen Stellung (vgl. z. B. Fig. 1, 4 und 7) und einer die jeweilige Fensteröffnung 7 überquerenden, vorgeschobenen Endstellung (vgl. z. B. Fig. 6, Fig. 9 und Fig. 10) verschiebbar. Sowohl in ihren vorgeschobenen Endstellungen als auch in ihren die Fensteröffnungen 7 freigebenden zurückgezogenen Stellungen befinden sich die Hohlnadeln 10 über einen Teil ihrer Längserstreckungen in den Bohrungen 8. In den zurückgezogenen Stellungen ragen die vorderen Enden der Hohlnadeln 10 nicht aus den Bohrungen 8 heraus.

In diesem Ausführungsbeispiel sind die Hohlnadeln 10 zwischen ihrer zurückgezogenen Stellung und ihrer vorgeschobenen Endstellung mittels einer Kulissenführung 11 verschiebbar. Hierzu sind die mit einem hinteren Endabschnitt je nach Verschiebestellung mehr oder weniger weit aus den Bohrungen 8 ragenden Hohlnadeln 10 an ihren hinteren Enden an einem Nadelhaltering 12 angebracht. Der Nadelhaltering 12 weist hierzu in achsialer Richtung verlaufende Bohrungen auf, in die die Enden der Hohlnadeln 10 eingesteckt sind und in denen sie befestigt sind, beispielsweise durch Verklebungen.

Der Nadelhaltering 12 ist im Abschnitt 9 des Tubus 1 angeordnet und weist nach außen abstehende Kulissenzapfen 13 auf, die im Abschnitt 9 angeordnete, in achsialer Richtung des Tubus 1 verlaufende Schlitze 14 durchsetzen. Die Kulissenzapfen 13 greifen in Kulissen 15 ein, die im Betätigungsring 16 angeordnet sind, der den Abschnitt 9 umgibt. Durch Verdrehen des Betätigungsrings 16 werden die Kulissenzapfen 13 von den schräg verlaufenden Kulissen 15 in achsialer Richtung verschoben (wobei der Nadelhaltering 12 durch die Schlitze 14 gegen eine Verdrehung gesichert ist), wodurch der Nadelhaltering 12 und mit ihm die Hohlnadeln 10 in achsialer Richtung des Tubus 1 verschoben werden.

Verschiebeeinrichtungen zur gleichzeitigen Verschiebung der beiden Hohlnadeln 10 in achsialer Richtung könnten auch in anderer Weise ausgebildet sein, beispielsweise über Kurvenscheiben, Kniehebel oder Spindelmuttergetriebe (= Schraubübersetzungen).

In der vorgeschobenen Endstellung der Hohlnadeln 10, in welcher diese die Fensteröffnungen 7 überqueren (vgl. Fig. 6, 9 und 10), wird ein durchgehender Führungskanal zum Durchführen eines Fadens ausgebildet, wie weiter unten noch genauer erläutert wird. Die inneren Kanäle 17 der Hohlnadeln 10 bilden jeweils einen Abschnitt dieses Führungskanals. Der Führungskanal umfasst weiters einen gekrümmt verlaufenden Verbindungsabschnitt 18, der die vorderen Enden der inneren Kanäle 17 der Hohlnadeln 10 in deren vorderen Endstellungen verbindet.

In diesem Ausführungsbeispiel verläuft zur Ausbildung dieses Verbindungsabschnitts 18 über die den Tubus 1 vorne verschließende Stirnwand eine Nut 19, die sich bis zu den beiden gegenüberliegenden Fensteröffnungen 7 erstreckt und in diese mündet.

Die in diesem Ausführungsbeispiel relativ tief ausgebildete Nut 19 besitzt über die Tiefe der Nut 19 gesehen einen breiteren, an die äußere Oberfläche des Tubus 1 anschließenden Abschnitt, an den über eine Stufe 20 ein Abschnitt mit einer verringerten Nutbreite anschließt, an welchen über eine Stufe 21 ein Abschnitt mit einer wiederum breiteren Nutbreite anschließt, der den Verbindungsabschnitt 18 bildet. Der Verbindungsabschnitt 18 des Führungskanals wird zum inneren Hohlraum 6 des Tubus 1 von einer Zwischenwand 22 abgegrenzt, deren den Verbindungsabschnitt 18 begrenzende Oberfläche den Nutgrund der Nut 19 darstellt.

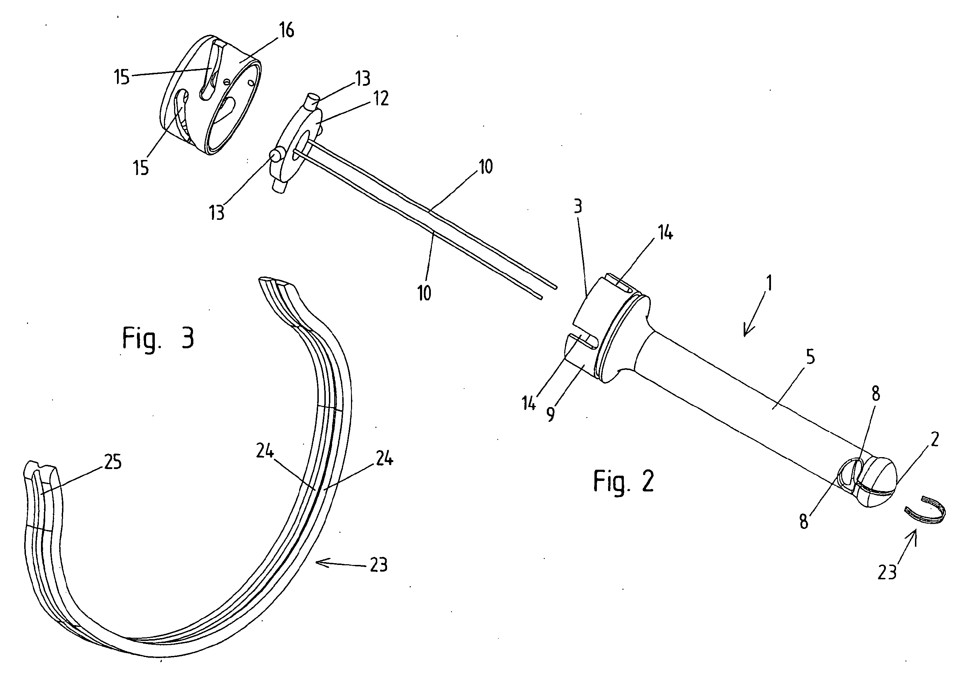

Im an die äußere Oberfläche des Tubus 1 anschließenden verbreiterten Abschnitt der Nut 19 ist eine Verschlusseinrichtung 23 angeordnet, die den Verbindungsabschnitt 18 über seine gesamte Längserstreckung abdeckt und somit zum den Tubus 1 umgebenden Außenraum schließt. Diese Verschlusseinrichtung wird hier von einem in Fig. 3 vergrößert dargestellten folienartigen Streifen aus einem Kunststoffmaterial gebildet. Der Streifen weist über seine gesamte Längserstreckung beidseitig verlaufende dickere Bereiche und einen dazwischenliegenden dünneren Bereich auf, der eine Art Sollbruchstelle des Streifens bildet, wie weiter unten noch genauer erläutert wird. Der Streifen ist mit seinen dickeren Bereichen an den Stufen 20 angeklebt.

Der Tubus 1 weist im gezeigten Ausführungsbeispiel einen an die Fensteröffnungen 7 zum Einsteckende 2 hin anschließenden vergrößerten Kopf auf, der das Einsteckende 2 bzw. die vordere Stirnseite des Tubus 1 bildet. Es wäre auch denkbar und möglich, dass der Tubus 1 bis zu seinem abgerundeten oder in Form eines Kegels mit einer abgerundeten Spitze ausgebildeten Ende den gleichen Außendurchmesser beibehält.

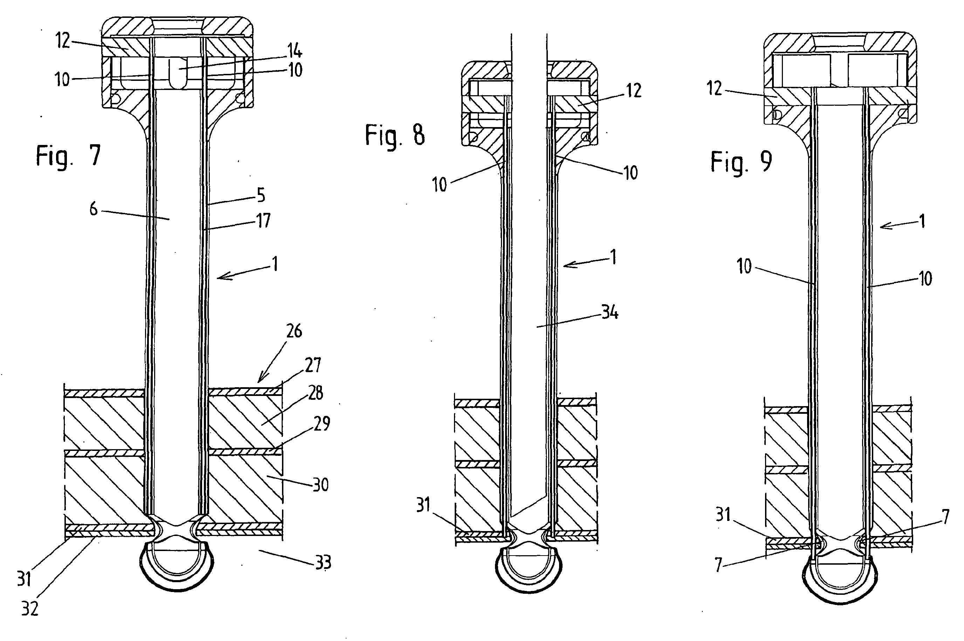

Die Durchführung eines Fascienverschlusses durch Anbringung einer Naht mittels einer erfindungsgemäßen Einrichtung wird im Folgenden anhand der Fig. 7 bis 13 erläutert. Der Tubus 1 wird zunächst in den Stichkanal in der Bauchdecke 26 eingeführt, wie dies in Fig. 7 dargestellt ist. Der Aufbau der Bauchdecke 26 ist schematisch dargestellt. An die Haut 27 schließt die Fettschicht 28 an, die von einer Fascie 29 von der darunterliegenden Muskelschicht 30 abgegrenzt ist. Unterhalb der Muskelschicht 30 liegt die posteriore Fascie 31, die für die Stabilität der Bauchdecke von wesentlicher Bedeutung ist. Unterhalb der Fascie 31 schließt das Bauchfell 32 an, welches den Bauchraum 33 begrenzt.

Der Tubus 1 wird soweit in den Stichkanal eingeführt, dass sich die Fascie 31 im Bereich der Fensteröffnungen 7 befindet. Die Hohlnadeln befinden sich in ihren die Fensteröffnungen 7 freigebenden zurückgezogenen Stellungen. Durch die Eigenspannung des Gewebes wölbt sich dieses in die Fensteröffnungen 7 hinein. Wenn dies gewünscht ist, so kann das Gewebe weiter in die Fensteröffnungen 7 hineingezogen werden, indem im Hohlraum 6 des Tubus 1 ein Unterdruck erzeugt wird (mittels einer entsprechenden Abpumpeinrichtung).

Als nächstes werden die Hohlnadeln 10 nach vorne geschoben. In Fig. 8 ist eine Zwischenstellung dargestellt. Das in die Fensteröffnungen 7 eingefallene Gewebe wird hierbei von den Hohlnadeln 10 durchstochen. Dieser Vorgang kann durch ein in den inneren Hohlraum 6 des Tubus 1 eingeschobenes Endoskop 34 optisch kontrolliert werden, wie dies in Fig. 8 ebenfalls schematisch dargestellt ist.

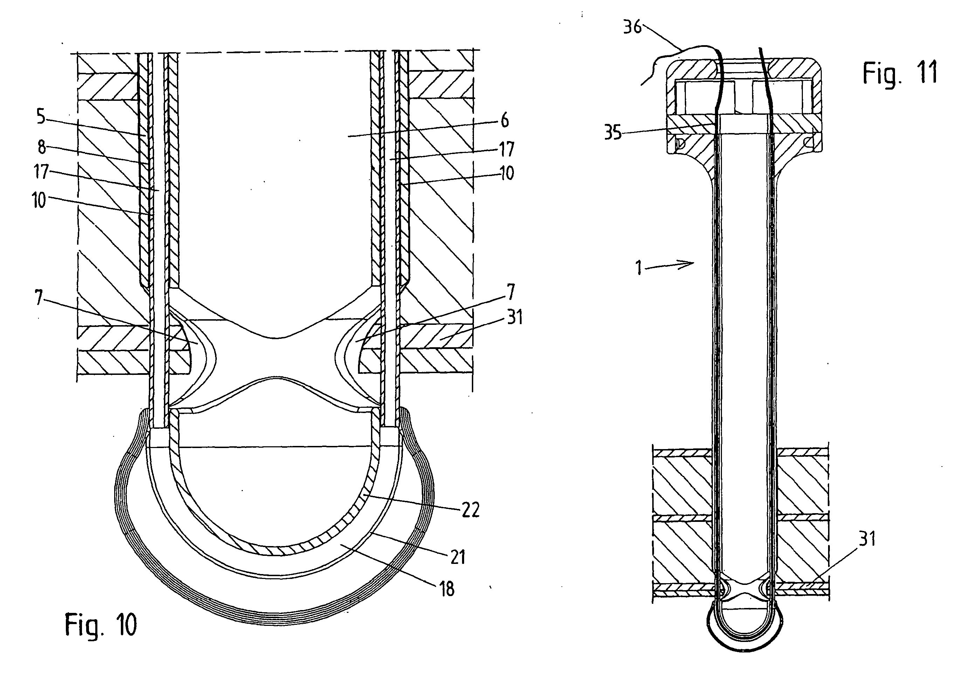

In Fig. 9 ist der Zustand dargestellt, wenn die Hohlnadeln 10 in ihre die Fensteröffnungen 7 überquerenden Endstellungen vorgeschoben worden sind. Die inneren Kanäle 17 der Hohlnadeln 10 bilden zusammen mit dem Verbindungsabschnitt 18 einen durchgehenden Führungskanal. Fig. 10 zeigt einen vergrößerten Ausschnitt der Darstellung von Fig. 9.

In der Folge wird durch diesen Führungskanal ein Fadeneinzugselement durchgesteckt, wie dies in Fig. 11 dargestellt ist. Am einen Ende des Fadeneinzugselements 35 ist hierbei ein Faden 36 befestigt. Das Fadeneinzugselement 35, das eine größere Länge als der Führungskanal aufweist, kann beispielsweise von einem flexiblen Draht gebildet werden. Auch die Ausbildung in Form einer Schraubenfeder, deren Durchmesser kleiner als der Durchmesser des Führungskanals ist, ist denkbar und möglich. Im ungebogenen Zustand der Schraubenfeder können hierbei die einzelnen Windungen unmittelbar aneinander anliegen.

Die Dicke des Fadeneinzugselements 35 ist größer als die Breite des zwischen den Stufen 20, 21 sich erstreckenden Abschnitts der Nut 19. Das Fadeneinzugselement 35 kann somit nicht aus dem Verbindungsabschnitt 18 austreten, sondern wird, wenn es aus dem vorderen Ende des inneren Kanals der einen Hohlnadel 10 austritt, vom gekrümmten Führungskanal geführt, bis es wieder in den inneren Kanal der anderen Hohlnadel 10 eintritt.

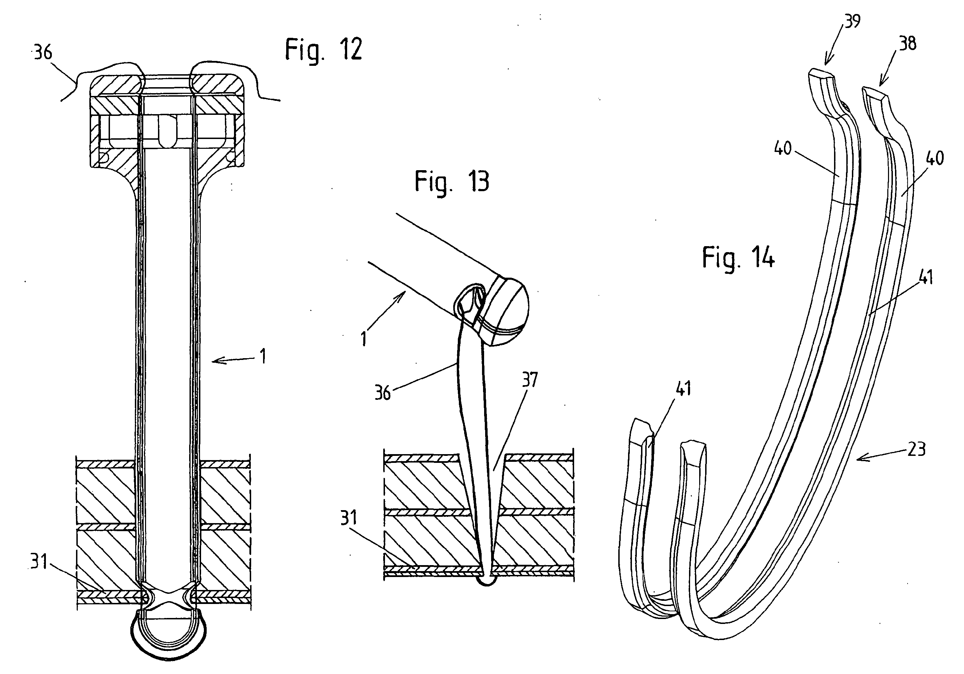

In der Folge wird das Fadeneinzugselement 35 mit dem anderen Ende aus dem Führungskanal herausgezogen, wodurch der Faden 36 durch den Führungskanal durchgefädelt wird, wie dies aus Fig. 12 ersichtlich ist.

In der Folge werden die Hohlnadeln 10 wiederum in ihre die Fensteröffnungen 7 freigebenden Stellungen zurückgezogen und der Tubus 1 wird aus dem Stichkanal 37 herausgezogen. Der Stichkanal 37 ist in Fig. 13 nur der Übersichtlichkeit halber geöffnet dargestellt, tatsächlich wird er sich nach dem Herausziehen des Tubus 1 schließen.

Beim Herausziehen des Tubus 1 aus dem Stichkanal wird der Faden 36, der beidseitig des Stichkanals die Fascie 31 durchsetzt (und einen Abschnitt der anschließenden Muskelschicht 30) von diesem Körpergewebe zurückgehalten und der zuvor im Verbindungsabschnitt 18 liegende Abschnitt des Fadens 36 gelangt aus dem Verbindungsabschnitt 18 unter Durchführung durch den zwischen den Schultern 20, 21 liegenden schmaleren Abschnitt der Nut 19 und Durchreißen der Verschlusseinrichtung 23 durch deren dünneren Bereich 25 in den außerhalb des Tubus 1 liegenden Außenraum. Weiters wird der Faden aus den inneren Kanälen 17 der Hohlnadeln nach vorne herausgezogen. Dies ist in Fig. 13 dargestellt. Die Naht ist somit vorgelegt und die Fadenenden können nunmehr zur Ausbildung der Naht verknüpft werden.

Eine weitere mögliche Ausbildungsform der Verschlusseinrichtung ist in Fig. 14 dargestellt. Die Verschlusseinrichtung ist hier von vorne herein zweigeteilt ausgebildet und besitzt zwei Streifenabschnitte 38, 39, die jeweils einen über die Länge des Streifenabschnitts 38, 39 verlaufenden dickeren Bereich 40 und einen dünneren vorspringenden Steg 41 aufweisen. Die freien Stirnseiten der Stege 41 liegen im in die Nut 19 eingesetzten Zustand der Verschlusseinrichtung, in welchem ein jeweiliger Streifenabschnitt 38, 39 an einer der Stufen 20 angeklebt ist, über die Länge der Nut 19 aneinander an. Die Auszugskraft für den Faden 36 ist bei dieser Verschlusseinrichtung verringert.

Denkbar und möglich wäre es auch, dass der Verbindungsabschnitt 18 des Führungskanals offen bleibt. Der an den Verbindungsabschnitt 18 des Führungskanals zur äußeren Oberfläche des Tubus hin anschließende schmalere Abschnitt der Nut 19 könnte sich in diesem Fall bis zur äußeren Oberfläche des Tubus 1 erstrecken (d. h. die Stufe 20 und der nach außen anschließende breitere Abschnitt der Nut 19 würden entfallen). Bevorzugterweise ist aber eine Verschlusseinrichtung für den Verbindungsabschnitt des Führungskanals vorhanden. Denkbar und möglich wäre es auch, diese in Form eines mechanischen, öffen- und schließbaren Verschlusses auszuführen. Beispielsweise könnte ein in Richtung quer zum Verbindungsabschnitt am oder im Tubus verschiebbar gelagertes Teil zwischen einer den Verbindungsabschnitt überdeckenden und den Verbindungsabschnitt freigebenden Stellung durch ein Betätigungsmittel verschiebbar sein.

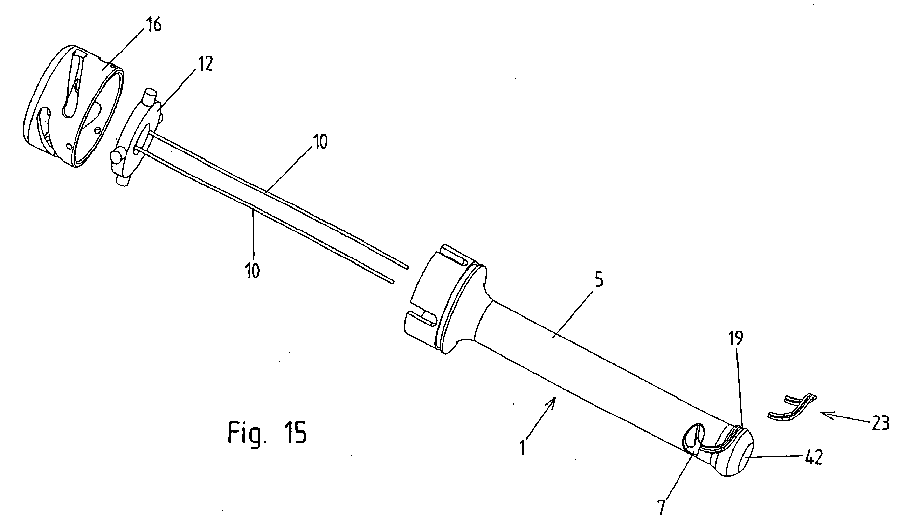

Eine weitere Ausführungsform einer erfindungsgemäßen Einrichtung ist in den Fig. 15 bis 17 dargestellt. Abgesehen von den im Folgenden beschriebenen Änderungen entspricht diese Ausführungsform der bereits anhand der Fig. 1 bis 13 beschriebenen Ausführungsform und analoge Teile wurden mit den gleichen Bezugszeichen versehen.

Im Unterschied zur Ausführungsform gemäß den Fig. 1 bis 13 besitzt der Tubus (= Hülse) 1 an seinem vorderen Ende eine Öffnung 42. Zur Ausbildung des Verbindungsabschnittes 18 des Führungskanals ist wiederum eine Nut 19 in die äußere Oberfläche des Tubus 1 eingebracht, die nunmehr aber über die Mantelwand des Tubus 1 verläuft, sodass der Verbindungsabschnitt 18 innerhalb der Mantelwand 5 des Tubus 1 verläuft. Der Verbindungsabschnitt 18 besitzt hierbei ausgehend von den Fensteröffnungen 7 nach vorne in Richtung zum Einsteckende 2 verlaufende Abschnitte, die bogenförmig in einen diese beiden nach vorne verlaufenden Abschnitte verbindenden, in Umfangsrichtung verlaufenden Abschnitt übergehen.

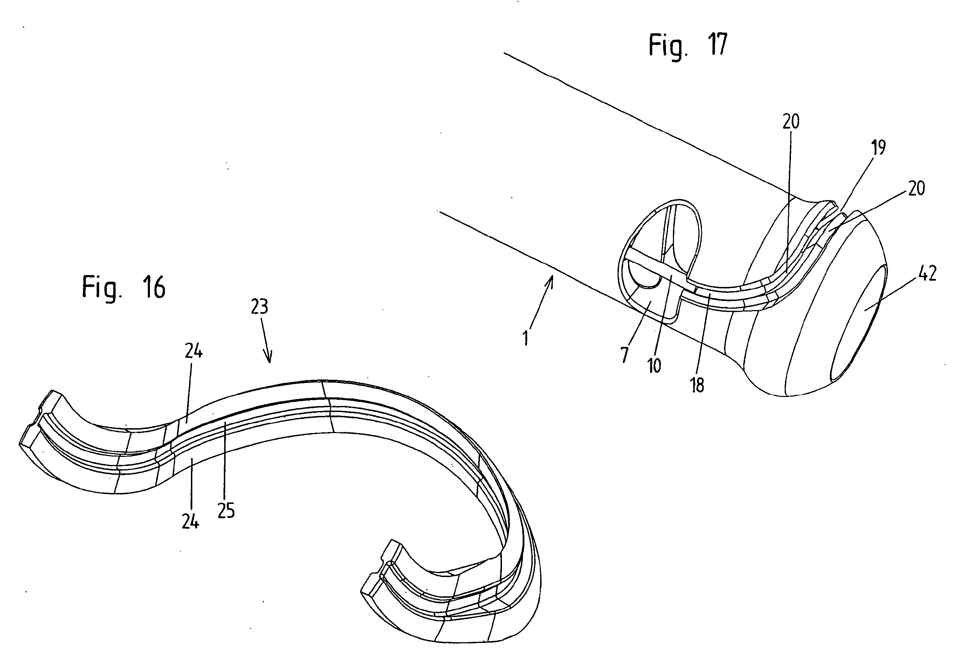

Die Verschlusseinrichtung 23 besitzt eine an diesen Verlauf des Verbindungsabschnitts 18 bzw. der Nut 19 angepasste gekrümmte Form, wie dies in Fig. 16 dargestellt ist. Wiederum liegt zwischen über die Länge der Verschlusseinrichtung verlaufenden dickeren Bereichen 24 ein mittlerer dünnerer Bereich 25, der eine Sollbruchstelle zum Durchreißen des Fadens 36 darstellt. Die Verschlusseinrichtung könnte auch analog zu der in Fig. 14 dargestellten Verschlusseinrichtung zweiteilig ausgebildet sein.

Aufgrund der vorderen Öffnung 42 des Tubus 1, die den inneren Hohlraum 6 fortsetzt, kann die Einrichtung gleichzeitig als Trokar ausgebildet sein. Hierzu weiters erforderliche Trokarbauteile, wie Ventile und/oder Dichtungen sind in Fig. 15 der Übersichtlichkeit halber nicht dargestellt. Solche Trokarbauteile sind bekannt und müssen in dieser Schrift nicht weiter erläutert werden.

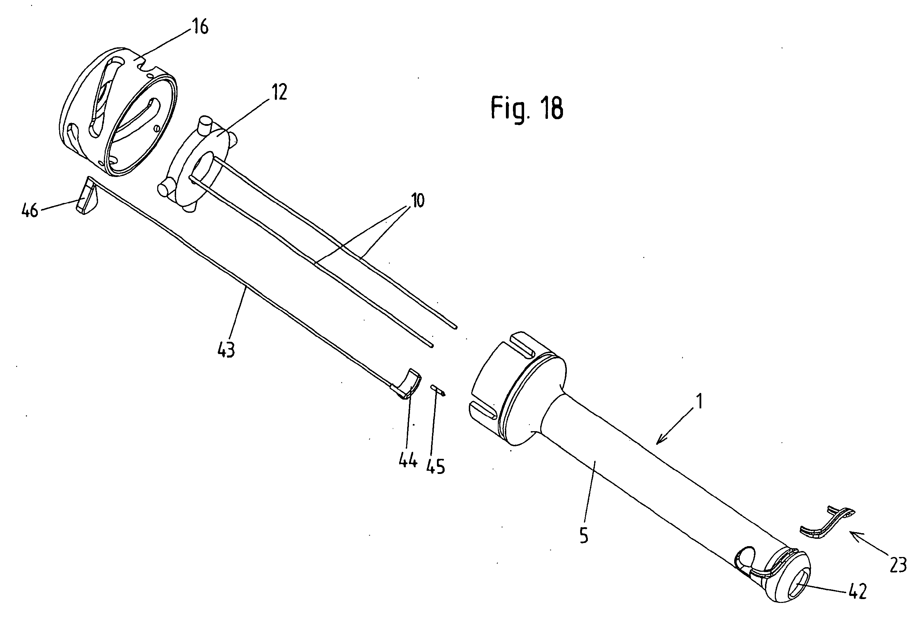

Die in den Fig. 18 bis 20 dargestellte Ausführungsform entspricht der in den Fig. 15 bis 17 dargestellten Ausführungsform abgesehen von folgenden Änderungen:

In der Mantelwand 5 des Tubus 1 ist eine weitere in Längsrichtung verlaufende Bohrung eingebracht, welche von einer Welle 44 durchsetzt wird. Am vorderen Ende der Welle ist ein Halter 44 für ein Schneidwerkzeug 45 angebracht. Am hinteren Ende ist ein Betätigungsteil 46 zum Verdrehen der Welle 43 und Verschwenken des Halters 44 angebracht.

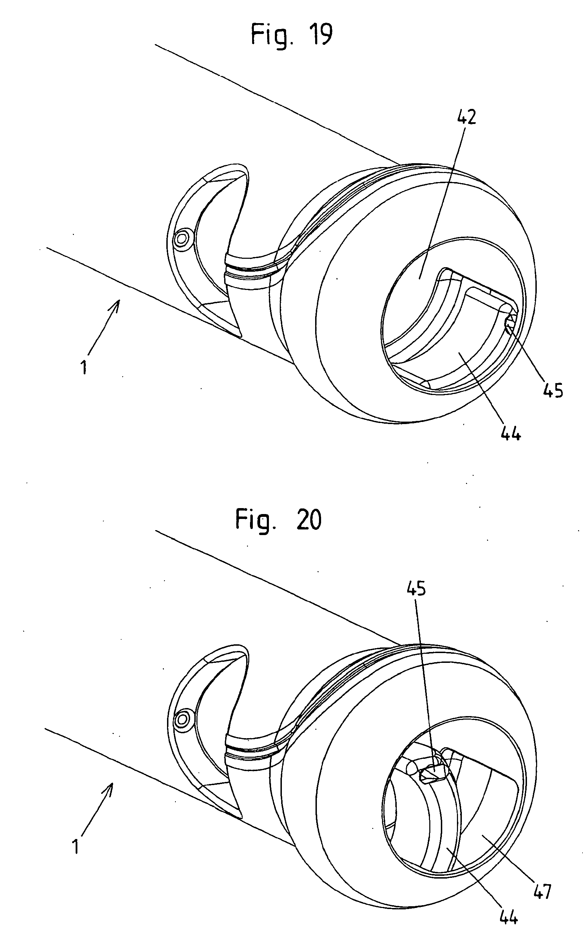

In der Grundstellung liegt der Halter 44 mit dem Schneidwerkzeug 45 in einer Vertiefung 47 in der inneren Mantelfläche des Tubus 1, wie dies in Fig. 19 dargestellt ist. Durch das Verdrehen der Welle 43 wird der Halter 44 mit dem Schneidwerkzeug 45 aus der Vertiefung 47 herausgeschwenkt, wie dies in Fig. 20 dargestellt ist. Das Schneidwerkzeug 45 befindet sich hierbei nahe beim Einsteckende 22 des Tubus 1 im Bereich der Öffnung 42. Wenn der Halter 44 in dieser Position durch Hin- und Herdrehen der Welle hin- und hergeschwenkt wird, wobei die Einrichtung auf die Bauchdecke des Patienten aufgedrückt wird, kann ein Stichkanal für den Tubus 1 ausgebildet werden, um diesen durch die Gewebsschichten in den Bauchraum einzuführen.

Die optische Überwachung der Platzierung des Tubus im Stichkanal und des Durchstichs der posterioren Fascie mittels der Hohlnadeln kann jeweils durch ein in den Hohlraum 6 des Tubus eingeführtes Endoskop erfolgen.

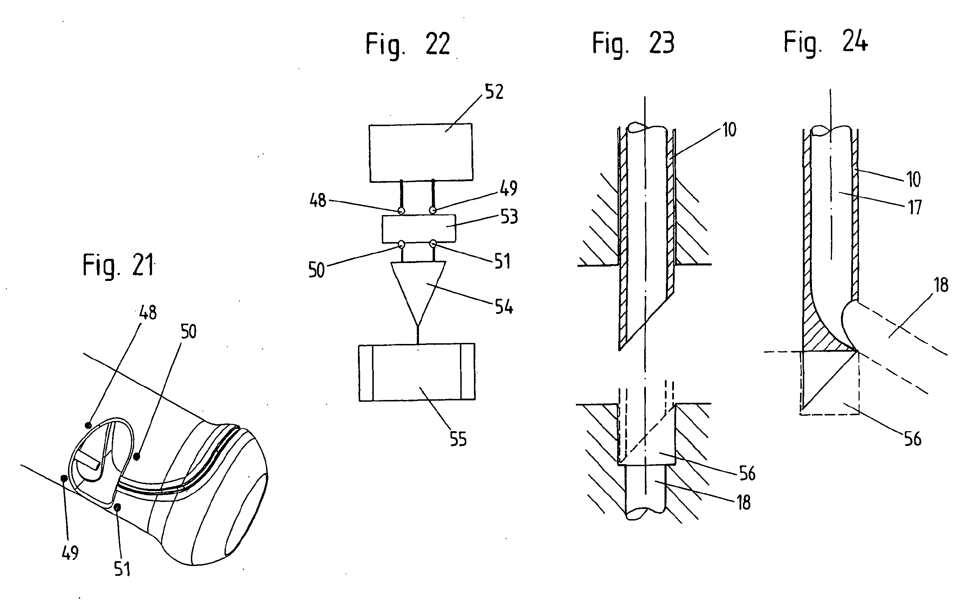

Zusätzlich kann in einer weiteren, in den Fig. 21 und 22 schematisch dargestellten Ausführungsform der Erfindung eine Überwachung der Platzierung des Tubus mittels einer Impedanzmessung erfolgen. Hierzu sind an der Mantelwand des Tubus Elektroden 48 bis 51 angeordnet. Über zwei hinter einer der Fensteröffnungen 7 beabstandet angeordnete Elektroden 49, 50 wird ein stromkonstanter Messimpuls in das Gewebe eingeprägt und über zwei vor dieser Fensteröffnung 7 angeordnete beabstandete Elektroden 50, 51 wird der resultierende Spannungsabfall gemessen. Dies ermöglicht die Bestimmung der Gewebsimpedanz, wodurch eine Klassifizierung der Gewebsart, die an den Elektroden anliegt, durchführbar ist. Nach der Gewebserkennung kann die korrekte Einstichposition dem Chirurgen beispielsweise über eine optische Anzeige signalisiert werden, wodurch diese Technik eine Ergänzung oder eine Alternative zur Sichtkontrolle beim Setzen der Naht darstellt. Fig. 22 zeigt ein Blockdiagramm der Anordnung. Der Stromfluss zwischen den Elektroden 48, 49 wird durch die Wechselstromquelle 52 hervorgerufen. Das dazwischenliegende Gewebe 53 ist schematisch dargestellt. Die Spannung zwischen den Elektroden 50, 51 wird mittels eines Verstärkers 54 verstärkt und einer Auswert- und Anzeigeeinheit 55 zugeführt.

Fig. 23 zeigt eine schematische Darstellung einer möglichen Ausbildung eines Gegenlagers für eine jeweilige Hohlnadel 10, welche beispielsweise an ihrem vorderen Ende abgeschrägt ausgebildet sein kann. An der der Austrittstelle der Hohlnadel 10 aus der Bohrung 8 gegenüberliegenden Seite ist eine Ausnehmung 56 angeordnet, welche vorteilhafterweise mit einer Einlaufschräge (= einem Einlauftrichter) ausgebildet ist. Beim Vorschieben der Hohlnadel 10 in ihre vordere Endstellung wird das vordere Ende der Hohlnadel 10 in die Ausnehmung 56 eingefahren und dadurch zentriert. Vom Boden der Ausnehmung 56 geht der Verbindungsabschnitt 18 aus, vorzugsweise im Wesentlichen bündig zum inneren Kanal der Hohlnadel 10, während die Ausnehmung 56 demgegenüber breiter ist.

Durch das vorzugsweise vorgesehene abgeschrägte Ende der Hohlnadel wird beim Durchstechen des Gewebes eine nach innen gerichtete Kraftkomponente hervorgerufen, wodurch das Gewebe weiter in die Fensteröffnung 7 hineingedrückt wird. Ein solches abgeschrägtes Ende der Hohlnadel ist auch für die anderen beschriebenen Ausführungsbeispiele bevorzugt (obwohl es in den Fig. 1 bis 19 nicht dargestellt ist).

Bei der in Fig. 24 schematisch dargestellten Ausführungsform der Hohlnadel 10 tritt der innere Kanal 17 der Hohlnadel in der Nähe der Spitze der Hohlnadel seitlich aus dieser aus. Der Verbindungsabschnitt 18, der in Fig. 24 lediglich durch strichlierte Linien angedeutet ist, ist in seiner Platzierung und Ausrichtung entsprechend angepasst. Die Spitze der Hohlnadel ist vorzugsweise wiederum abgeschrägt ausgebildet und wird in der vorgeschobenen Endstellung der Hohlnadel von der sacklochartigen Ausnehmung 56 aufgenommen, die ein Gegenlager für die Hohlnadel 10 bildet.

Insbesondere zum Verschließen von großen Trokareinstichen, beispielsweise für Trokargrößen von 15mm und mehr, könnten auch jeder Fensteröffnung 7 zwei an ihrem vom Einsteckende abgelegenen Rand mündende Bohrungen in der Mantelwand 5 zugeordnet sein, in welchen jeweils eine in Längsrichtung verschiebbar Hohlnadel angeordnet ist. Es wären dann weiters auch zwei Verbindungsabschnitte 18 vorzusehen, welche jeweils die inneren Kanäle von zwei Hohlnadeln 10 verbinden, die gegenüberliegende Fensteröffnungen in ihrer vorderen Endstellung überqueren. Auf diese Weise könnten zwei die posteriore Fascie verschließende Einzelknopfnähte gelegt werden. Vorzugsweise können hierbei alle vier Hohlnadeln durch eine gemeinsame Verschiebeeinrichtung gleichzeitig in Längsrichtung des Tubus 1 verschoben werden.

Unterschiedliche weitere Modifikationen der gezeigten Ausführungsbeispiele der Erfindung sind denkbar und möglich, ohne den Bereich der Erfindung zu verlassen. So wären beispielsweise auch Ausbildungen des Tubus 1 denkbar und möglich, bei welchen dieser keinen hinteren Abschnitt 9 mit einem vergrößerten Durchmesser aufweist. Die Bohrungen 8 würden sich in diesem Fall bis zum hinteren Ende des Tubus erstrecken und dort aus der Mantelwand austreten. Es könnte in diesem Fall hinter dem Tubus eine Einrichtung zur Längsverschiebung der Hohlnadeln angeordnet werden, die beispielsweise mit einer Halterung für den Tubus verbunden ist.

### Legende zu den Hinweisziffern:

- 1: Tubus
- 2: Einsteckende
- 3: hinteres Ende
- 4: Längsachse
- 5: Mantelwand
- 6: Hohlraum
- 7: Fensteröffnung
- 8: Bohrung
- 9: Abschnitt
- 10: Hohlnadel
- 11: Kulissenführung
- 12: Nadelhaltering
- 13: Kulissenzapfen
- 14: Schlitz
- 15: Kulisse
- 16: Betätigungsring
- 17: innerer Kanal
- 18: Verbindungsabschnitt
- 19: Nut
- 20: Stufe
- 21: Stufe
- 22: Zwischenwand
- 23: Verschlusseinrichtung
- 24: dickerer Bereich
- 25: dünnerer Bereich
- 26: Bauchdecke
- 27: Haut
- 28: Fettschicht
- 29: Fascie
- 30: Muskelschicht
- 31: posteriore Fascie
- 32: Bauchfell
- 33: Bauchraum
- 34: Endoskop
- 35: Fadeneinzugselement
- 36: Faden
- 37: Stichkanal
- 38: Streifenabschnitt
- 39: Streifenabschnitt
- 40: dickerer Bereich
- 41: Steg
- 42: Öffnung
- 43: Welle
- 44: Halter
- 45: Schneidewerkzeug
- 46: Betätigungsteil
- 47: Vertiefung
- 48: Elektrode
- 49: Elektrode
- 50: Elektrode
- 51: Elektrode
- 52: Wechselstromquelle
- 53: Gewebe
- 54: Verstärker
- 55: Auswert- und Anzeigeeinheit
- 56: Ausnehmung

## Patentansprüche

1. Einrichtung zum chirurgischen Verschluss eines bei einer laparoskopischen Operation eingebrachten Trokardurchstichs durch eine posteriore Fascie, welche einen Tubus (1) mit einem vorderen Einsteckende (2) und einem hinteren Ende (3) aufweist, in dessen Mantelwand (5) gegenüberliegende Fensteröffnungen (7) ausgebildet sind, in die an ihren vom Einsteckende (2) des Tubus (1) abgelegenen Rändern jeweils mindestens eine in der Mantelwand (5) in Längsrichtung des Tubus (1) verlaufende Bohrung (8) mündet, in welcher jeweils eine Nadel (10) der Einrichtung zwischen einer die jeweilige Fensteröffnung (7) freigebenden zurückgezogenen Stellung und einer die Fensteröffnung (7) überquerenden Endstellung verschiebbar ist, **dadurch gekennzeichnet, dass** die in den Bohrungen (8) der Mantelwand (5) des Tubus (1) verschiebbaren Nadeln als Hohlnadeln (10) ausgebildet sind, wobei die inneren Kanäle (17) der Hohlnadeln (10) jeweils einen Abschnitt eines Führungskanals zum Durchführen eines Fadens (36) bilden und dieser Führungskanal weiters mindestens einen gekrümmten Verbindungsabschnitt (18) aufweist, der die vorderen Enden der inneren Kanäle (17) von zwei sich in ihren Endstellungen befindenden und gegenüberliegende Fensteröffnungen (7) überquerenden Hohlnadeln (10) verbindet und der über seine gesamte Längserstreckung zum den Tubus (1) umgebenden Außenraum von einer öffenbaren Verschlusseinrichtung (23) abgedeckt ist oder mit dem den Tubus (1) umgebenden Außenraum über einen Schlitz verbunden ist, wobei ein im Verbindungsabschnitt (18) sich befindender Abschnitt des Fadens (36) unter Öffnung der Verschlusseinrichtung (23) oder durch den Schlitz aus dem Verbindungsabschnitt (18) des Führungskanals in den Außenraum herausbringbar ist.

2. Einrichtung nach Anspruch 1, **dadurch gekennzeichnet, dass** die öffenbare Verschlusseinrichtung (23) von einem vom Faden (36) durchreißbaren oder einseitig vom Tubus (1) abreißbaren Streifen gebildet wird.

3. Einrichtung nach Anspruch 1 oder Anspruch 2, **dadurch gekennzeichnet, dass** die Fensteröffnungen (7) in einem an das Einsteckende (2) anschließenden vorderen Endabschnitt des Tubus (1), vorzugsweise im vorderen Viertel der Längserstreckung des Tubus (1), angeordnet sind.

4. Einrichtung nach einem der Ansprüche 1 bis 3, **dadurch gekennzeichnet, dass** nur zwei gegenüberliegende Fensteröffnungen (7) vorhanden sind, die sich zusammen vorzugsweise jeweils zumindest über 30% des Umfangs des Tubus (1) erstrecken.

5. Einrichtung nach einem der Ansprüche 1 bis 4, **dadurch gekennzeichnet, dass** sich die Hohlnadeln (10) sowohl in ihren die Fensteröffnungen (7) freigebenden zurückgezogenen Stellungen als auch in ihren vorgeschobenen Endstellungen zumindest über einen Teil ihrer Längserstreckungen in den Bohrungen (8) befinden.

6. Einrichtung nach einem der Ansprüche 1 bis 5, **dadurch gekennzeichnet, dass** die Bohrungen (10) in der Mantelwand (5) an einer zum hinteren Ende des Tubus (1) weisenden Stufe des Tubus (1), an welche zum hinteren Ende (3) ein hinterer erweiterter Abschnitt (9) des Tubus (1) anschließt, aus dem Tubus (1) herausführen.

7. Einrichtung nach einem der Ansprüche 1 bis 6, **dadurch gekennzeichnet, dass** eine Verschiebeeinrichtung zur gleichzeitigen Längsverschiebung der Hohlnadeln (10) vorhanden ist.

8. Einrichtung nach Anspruch 7, **dadurch gekennzeichnet, dass** die Verschiebeeinrichtung einen Nadelhaltering (12) umfasst, an dem die Hohlnadeln (10) im Bereich ihrer hinteren Enden angebracht sind.

9. Einrichtung nach Anspruch 8, **dadurch gekennzeichnet, dass** der Nadelhaltering (12) durch ein Verdrehen eines verdrehbaren Betätigungsrings (16) in Längsrichtung des Tubus (1) verschiebbar ist.

10. Einrichtung nach Anspruch 9, **dadurch gekennzeichnet, dass** der Nadelhaltering (12) im hinteren erweiterten Abschnitt (9) des Tubus (1) angeordnet ist.

11. Einrichtung nach Anspruch 10, **dadurch gekennzeichnet, dass** der Nadelhaltering (12) zu seiner Verschiebung in Längsrichtung des Tubus (1) nach außen abstehende Kulissenzapfen (13) aufweist, die in achsialer Richtung des Tubus (1) verlaufende Schlitze (14) im erweiterten Abschnitt (9) des Tubus (1) durchsetzen und in im Betätigungsring (16) angeordneten Kulissen (15) ragen.

12. Einrichtung nach einem der Ansprüche 1 bis 11, **dadurch gekennzeichnet, dass** die Hohlnadeln (10) sowohl in ihren die Fensteröffnungen (7) freigebenden zurückgezogenen Stellungen als auch in ihren die Fensteröffnungen (7) überquerenden Endstellungen aus den hinteren Enden der Bohrungen (8) herausragen.

13. Einrichtung nach einem der Ansprüche 1 bis 12, **dadurch gekennzeichnet, dass** die Hohlnadeln (10) in den vorgeschobenen Endstellungen mit vorderen Endabschnitten in von den Rändern der Fensteröffnungen (7), die vom hinteren Ende (3) des Tubus (1) abgelegen sind, ausgehende Ausnehmungen (56) ragen, welche Gegenlager bilden.

14. Einrichtung nach einem der Ansprüche 1 bis 13, **dadurch gekennzeichnet, dass** der Tubus (1) an seinem Einsteckende (2) durch eine Stirnwand geschlossen ist.

15. Einrichtung nach Anspruch 14, **dadurch gekennzeichnet, dass** der Verbindungsabschnitt (18) des Führungskanals durch die die vordere Stirnseite des Tubus (1) bildende Wand des Tubus (1) verläuft.

16. Einrichtung nach einem der Ansprüche 1 bis 13, **dadurch gekennzeichnet, dass** der Tubus (1) an seinem Einsteckende (2) eine Öffnung (42) aufweist.

17. Einrichtung nach Anspruch 16, **dadurch gekennzeichnet, dass** der Verbindungsabschnitt in einem zwischen den Fensteröffnungen (7) und dem Einsteckende (2) des Tubus (1) gelegenen Bereich des Tubus (1) in der Mantelwand (5) des Tubus (1) verläuft.

18. Einrichtung nach einem der Ansprüche 1 bis 17, **dadurch gekennzeichnet, dass** am Tubus (1) Elektroden (48, 49, 50, 51) zur Durchführung einer Impedanzmessung angeordnet sind, die vorzugsweise vor und hinter zumindest einer der Fensteröffnungen (7) liegen.

19. Einrichtung nach einem der Ansprüche 1 bis 18, **dadurch gekennzeichnet, dass** der Führungskanal in der vorgeschobenen Endstellung der Hohlnadeln (10) vor dem Öffnen der den Verbindungsabschnitt (18) abdeckenden Verschlusseinrichtung (23) über seine gesamte Längserstreckung zum den Tubus (1) umgebenden Außenraum geschlossen ist.

20. Einrichtung nach einem der Ansprüche 1 bis 19, **dadurch gekennzeichnet, dass** der Faden (36) mittels eines durch den Führungskanal durchschiebbaren und hierbei vom Führungskanal geführten Fadeneinzugselements (35), dessen Länge größer als die des Führungskanals ist, in den Führungskanal einziehbar ist.

## Claims

1. A device for the surgical closure of a trocar puncture, made during a laparoscopic operation, through a posterior fascie, having a tube (1) with a front insertion end (2) and a rear end (3), facing window openings (7) being formed in the casing wall (5) of the tube (1) and at least one bore (8) which runs in the longitudinal direction of the tube (1) in the casing wall (5) opening into a respective window opening (7) at its edge remote from the insertion end (2) of the tube (1), a respective needle (10) of the device being displaceable in the bore (8) between a retracted position in which it opens the respective window opening (7) and an end position in which it traverses the window opening (7), **characterised in that** the needles displaceable in the bores (8) of the casing wall (5) of the tube (1) are in the form of hollow needles (10), the inner channels (17) of the hollow needles (10) each forming a portion of a guide channel for the passage of a thread (36) and this guide channel furthermore having at least one curved connecting portion (18) which connects the front ends of the inner channels (17) of two hollow needles (10) located in their end positions and traversing facing window openings (7) and which over its entire longitudinal extension is covered with respect to the exterior space surrounding the tube (1) by an openable closing device (23) or is connected to the exterior space surrounding the tube (1) via a slot, wherein a portion, of the thread (36), located in the connecting portion (18) is able to be brought out of the connecting portion (18) of the guide channel into the exterior space, accompanied by opening of the closing device (23) or through the slot.

2. A device according to claim 1, **characterised in that** the openable closing device (23) is formed by a strip though which the thread (36) can tear or which can be tom away from the tube (1) at one side.

3. A device according to claim 1 or claim 2, **characterised in that** the window openings (7) are arranged in a front end-portion of the tube (1) adjacent to the insertion end (2), are preferably arranged in the front quarter of the longitudinal extension of the tube (1).

4. A device according to any one of claims 1 to 3, **characterised in that** only two facing window openings (7) are present, together these preferably extending over at least 30% of the circumference of the tube (1) in each case.

5. A device according to any one of claims 1 to 4, **characterised in that** both in their retracted position opening the window opening (7) and in their advanced end position, the hollow needles (10) are located in the bores (8) over at least part of their longitudinal extension.

6. A device according to any one of claims 1 to 5, **characterised in that** the bores (10 [sic - recte 8]) in the casing wall (5) run out of the tube (1) at a step, of the tube (1), directed towards the rear end of the tube (1), the step being adjoined towards the rear end (3) by a rear widened portion (9) of the tube (1).

7. A device according to any one of claims 1 to 6, **characterised in that** a displacing device is present for simultaneous longitudinal displacement of the hollow needles (10).

8. A device according to claim 7, **characterised in that** the displacing device comprises a needle holding ring (12) to which the hollow needles (10) are attached in the region of their rear ends.

9. A device according to claim 8, **characterised in that** the needle holding ring (12) is displaceable in the longitudinal direction of the tube (1) through twisting a rotatable operating ring (16).

10. A device according to claim 9, **characterised in that** the needle holding ring (12) is arranged in the rear widened portion (9) of the tube (1).

11. A device according to claim 10, **characterised in that** the needle holding ring (12) has outwards projecting link pins (13) for its displacement in the longitudinal direction of the tube (1), which link pins (13) pass though slots (14), running in the axial direction of the tube (1), in the widened portion (9) of the tube (1) and project into links (15) arranged in the operating ring (16).

12. A device according to any one of claims 1 to 11, **characterised in that** the hollow needles (10) project from the rear end of the bores (8) both in their retracted position opening the window opening (7) and their end position traversing the window opening (7).

13. A device according to any one of claims 1 to 12, **characterised in that** in the advanced end position, the front end-portions of the hollow needles (10) project into recesses (56) issuing from the edges, remote from the rear end (3) of the tube (1), of the window openings (7), which recesses (56) form counter bearings.

14. A device according to any one of claims 1 to 13, **characterised in that** the insertion end (2) of the tube (1) is closed by an end wall.

15. A device according to claim 14, **characterised in that** the connecting portion (18) of the guide channel runs through the wall, of the tube (1), forming the front face of the tube (1).

16. A device according to any one of claims 1 to 13, **characterised in that** the tube (1) has an opening (42) at its insertion end (2).

17. A device according to claim 16, **characterised in that** the connecting portion runs in the casing wall (5) of the tube (1) in a region of the latter located between the window openings (7) and the insertion end (2) of the tube (1).

18. A device according to any one of claims 1 to 17, **characterised in that** electrodes (48, 49, 50, 51) to measure impedance are arranged on the tube (1), preferably lie in front of and behind at least one of the window openings (7).

19. A device according to any one of claims 1 to 18, **characterised in that** in the advanced end position of the hollow needles (10), the guide channel is, before opening of the closing device (23) covering the connecting portion (18), closed over its entire longitudinal extension towards the exterior space surrounding the tube (1).

20. A device according to any one of claims 1 to 19, **characterised in that** the thread (36) can be drawn into the guide channel by means of a thread feed element (35) which can be pushed through the guide channel and hereby guided by the guide channel and which is longer than the guide channel.

## Revendications

1. Dispositif de fermeture chirurgicale d'une perforation par trocart introduit à travers une aponévrose postérieure pendant une opération de coelioscopie, qui présente un tube (1) comportant une extrémité d'introduction (2) antérieure et une extrémité postérieure (3), dont la paroi d'enveloppe (5) comporte des fenêtres opposées (7) dans lesquelles respectivement au moins un perçage (8) s'étendant dans la paroi d'enveloppe (5) dans la direction longitudinale du tube (1) débouche sur leurs bords éloignés de l'extrémité d'introduction (2) du tube (1), perçage à l'intérieur duquel respectivement une aiguille (10) du dispositif est mobile entre une position retirée dégageant la fenêtre (7) respective et une position finale traversant la fenêtre (7),
**caractérisé en ce que**
les aiguilles mobiles dans les perçages (8) de la paroi d'enveloppe (5) du tube (1) sont des aiguilles creuses (10), les canaux intérieurs (17) des aiguilles creuses (10) forment respectivement un segment d'un canal de guidage pour le passage d'un fil (36) et ce canal de guidage présente également au moins un segment de liaison (18) courbe, lequel relie les extrémités antérieures des canaux intérieurs (17) de deux aiguilles creuses (10), se trouvant à leurs positions finales et traversant les fenêtres opposées (7), et est recouvert sur toute son étendue longitudinale d'un dispositif de fermeture (23) par rapport à l'espace extérieur entourant le tube (1), ce dispositif pouvant s'ouvrir, ou est relié par une fente à l'espace extérieur entourant le tube (1), et on peut sortir un segment du fil (36), situé dans le segment de liaison (18), hors du segment de liaison (18) du canal de guidage dans l'espace extérieur en ouvrant le dispositif de fermeture (23) ou à travers la fente.

2. Dispositif selon la revendication 1,
**caractérisé en ce que**
le dispositif de fermeture (23) pouvant s'ouvrir est constitué par une bande pouvant être déchirée par le fil (36) ou arrachée unilatéralement par le tube (1).

3. Dispositif selon la revendication 1 ou la revendication 2,
**caractérisé en ce que**
les fenêtres (7) sont disposées dans un segment d'extrémité antérieure du tube (1) faisant suite à l'extrémité d'introduction (2), de préférence dans le quart antérieur de l'étendue longitudinale du tube (1).

4. Dispositif selon l'une des revendications 1 à 3,
**caractérisé en ce qu'**
il y a seulement deux fenêtres (7) opposées qui ensemble s'étendent de préférence au moins respectivement sur 30 % de la périphérie du tube (1).

5. Dispositif selon l'une des revendications 1 à 4,
**caractérisé en ce que**
les aiguilles creuses (10) aussi bien dans leurs positions retirées dégageant les fenêtres (7) que dans leurs positions finales avancées, se trouvent au moins sur une partie de leurs étendues longitudinales dans les perçages (8).

6. Dispositif selon l'une des revendications 1 à 5,
**caractérisé en ce que**
les perçages (8) dans la paroi d'enveloppe (5) conduisent hors du tube (1) à un niveau du tube (1) dirigé vers l'extrémité postérieure du tube (1), auquel un segment élargi postérieur (9) du tube (1) fait suite vers l'extrémité postérieure (3).

7. Dispositif selon l'une des revendications 1 à 6,
**caractérisé par**
un dispositif de déplacement pour le déplacement longitudinal simultané des aiguilles creuses (10).

8. Dispositif selon la revendication 7,
**caractérisé en ce que**
le dispositif de déplacement comprend une bague de maintien des aiguilles (12) logeant la zone des extrémités postérieures des aiguilles creuses (10).

9. Dispositif selon la revendication 8,
**caractérisé en ce que**
la bague de maintien des aiguilles (12) peut se déplacer dans la direction longitudinale du tube (1) sous l'effet d'une rotation d'une bague d'actionnement rotative (16).

10. Dispositif selon la revendication 9,
**caractérisé en ce que**
la bague de maintien des aiguilles (12) est disposée dans le segment élargi postérieur (9) du tube (1).

11. Dispositif selon la revendication 10,
**caractérisé en ce que**
la bague de maintien des aiguilles (12) comporte, pour son déplacement dans la direction longitudinale du tube (1), des tourillons coulissants (13) dirigés vers l'extérieur, qui traversent des fentes (14) s'étendant dans la direction axiale du tube (1) dans le segment élargi postérieur (9) du tube (1) et dépassent dans des coulisses (15) disposées dans la bague d'actionnement (16).

12. Dispositif selon l'une des revendications 1 à 11,
**caractérisé en ce que**
les aiguilles creuses (10) dépassent des extrémités postérieures des perçages (8) aussi bien à leurs positions retirées dégageant les fenêtres (7) qu'à leurs positions finales traversant les fenêtres (7).

13. Dispositif selon l'une des revendications 1 à 12,
**caractérisé en ce que**
les aiguilles creuses (10), aux positions finales avancées, dépassent par les segments d'extrémité antérieurs dans des creux (56), qui forment des butées, partant des bords des fenêtres (7) qui sont éloignés de l'extrémité postérieure (3) du tube (1).

14. Dispositif selon l'une des revendications 1 à 13,
**caractérisé en ce que**
le tube (1) est fermé par une paroi frontale à son extrémité d'introduction (2).

15. Dispositif selon la revendication 14,
**caractérisé en ce que**
le segment de liaison (18) du canal de guidage s'étend à travers la paroi du tube (1) formant la face frontale antérieure du tube (1).

16. Dispositif selon l'une des revendications 1 à 13,
**caractérisé en ce que**
le tube (1) comporte une ouverture (42) à son extrémité d'introduction (2).

17. Dispositif selon la revendication 16,
**caractérisé en ce que**
dans une zone du tube (1) située entre les fenêtres (7) et l'extrémité d'introduction (2) du tube (1), le segment de liaison s'étend dans la paroi d'enveloppe (5) du tube (1).

18. Dispositif selon l'une des revendications 1 à 17,
**caractérisé en ce que**
des électrodes (48, 49, 50, 51) disposées sur le tube (1) pour réaliser une mesure d'impédance sont situées de préférence en avant et en arriére d'au moins l'une des fenêtres (7).

19. Dispositif selon l'une des revendications 1 à 18,
**caractérisé en ce que**
le canal de guidage, à la position finale avancée des aiguilles creuses (10), avant l'ouverture du dispositif de fermeture (23) recouvrant le segment de liaison (18), est fermé sur toute son étendue longitudinale par rapport à l'espace extérieur entourant le tube (1).

20. Dispositif selon l'une des revendications 1 à 19,
**caractérisé en ce que**
le fil (36) peut être tiré dans le canal de guidage au moyen d'un élément de tirage du fil (35) mobile à travers le canal de guidage, guidé par le canal de guidage, et dont la longueur est supérieure à celle du canal de guidage.
